# EUROPÄISCHE PATENTSCHRIFT

(11) **EP 0 978 293 B1**
(45) Veröffentlichungstag und Bekanntmachung des Hinweises auf die Patenterteilung: **09.11.2005**
(21) Anmeldenummer: 99113011.3
(22) Anmeldetag: 06.07.1999
(51) Int. Cl.: A61N 5/06

(54) **Bestrahlungsgerät**
Irradiation apparatus
Appareil d'irradiation

(30) Priorität: 07.08.1998 DE 19835800
(43) Veröffentlichungstag der Anmeldung: 09.02.2000
(73) Patentinhaber: uwe GmbH, 73525 Schwäbisch Gmünd (DE)
(72) Erfinder: Heudorfer, Edgar, 73563 Mögglingen (DE)
(74) Vertreter: Wolf, Eckhard

(56) Entgegenhaltungen:
- WO-A-87/06146
- DE-A- 3 440 478
- DE-A- 3 544 833
- DE-A- 4 324 379
- DE-U- 8 625 588
- US-A- 4 754 146

## Beschreibung

Die Erfindung betrifft ein Bestrahlungsgerät, insbesondere ein Solarium, mit einer Liege, mit einer auf der Liege angeordneten, an ihrer Oberfläche eine vorzugsweise muldenartige Liegefläche bildenden transparenten Liegeplatte, mit einer Anzahl innerhalb der Liege angeordneten, nach oben zur Liegeplatte hin strahlenden UV-Lampen und mit einer zwischen der Liegeplatte und den UV-Lampen angeordneten transparenten Abdeckplatte, die einen Kühlluftkanal für die UV-Lampen begrenzt.

Die überwiegend aus Acrylglas bestehenden Liegeplatten weisen oft kleinflächige Liegeflächen auf und sind relativ dickwandig ausgebildet, damit sie die beim bestimmungsgemäßen Gebrauch auftretenden Belastungen aufnehmen können. Eine Verbesserung in dieser Hinsicht kann dadurch erzielt werden, daß die Liegeplatten von unten her abgestützt werden. Die hierfür notwendigen Abstützelemente sind jedoch im lichtdurchfluteten Bereich angeordnet und stellen daher eine Störung für den Lichtdurchtritt dar. Diese kann zu einer ungleichmäßigen Bräunung führen. Die zwischen der Liegeplatte und den UV-Lampen angeordnete transparente Abdeckplatte dient vor allem der Luftführung. Sie soll sicherstellen, daß die Liegeplatte durch die UV-Lampen nicht unerwünscht aufgeheizt wird.

Aus der Druckschrift DE-A-4 324 379 ist ein Bestrahlungsgerät gemäß dem Oberbegriff des Anspruchs 1 bekannt.

Der Erfindung liegt die Aufgabe zugrunde, ein Bestrahlungsgerät der eingangs angegebenen Art zu entwickeln, bei dem trotz Verwendung großer Liegeflächen keine Störelemente vorhanden sind, die zu einer Variation in der Lichtdurchlässigkeit und zu einem ungleichmäßigen Bräunungsvorgang führen könnten. Ferner soll in Wartungsfalle ein einfacher Zugriff zu den UV-Lampen möglich sein.

Zur Lösung dieser Aufgaben wird die im Anspruch 1 angegebene Merkmalskombination vorgeschlagen. Vorteilhafte Ausgestaltungen und Weiterbildungen der Erfindung ergeben sich aus den Unteransprüchen.

Die erfindungsgemäße Lösung geht von dem Gedanken aus, daß die Abdeckplatte eine wellige Gestalt mit innerhalb der Liege nach oben und nach unten weisenden Wellenbögen aufweist, daß die Abdeckplatte sich zumindest mit einem Teil ihrer unteren Wellenbögen an Widerlagerflächen oder Widerlagerstegen der Liege abstützt, und daß die Liegeplatte mit ihrer Unterseite zumindest auf einem Teil der oberen Wellenbögen der Abdeckplatte aufliegt. Mit diesen Maßnahmen wird erreicht, daß es zwischen den UV-Lampen und der Liegeplatte im lichtdurchfluteten Bereich zu keinen Störungen im Lichtdurchtritt kommt.

Eine weitere Verbesserung in bezug auf die gleichmäßige Ausleuchtung des Bestrahlungsfeldes wird erzielt, wenn die Liegeplatte und/oder die Abdeckplatte aus mattiertem oder aufgerauhtem Acrylglas besteht. Mit dieser Maßnahme wird außerdem erreicht, daß die in der Liege befindlichen UV-Lampen durch die Liegeplatte und die Abdeckplatte hindurch als solche nicht sichtbar sind. Die Liegeplatte und/oder die Abdeckplatte können im Bereich ihrer fuß- und kopfseitigen Enden so undurchsichtig mattiert oder bedruckt werden, daß die darunter befindlichen Lampenfassungen von außen her nicht sichtbar sind.

Vorteilhafterweise ist den zweckmäßig als Leuchtstoffröhren ausgebildeten UV-Lampen jeweils ein quer zu deren Längserstreckung geschwungener Wellenbogen innerhalb der Abdeckplatte zugeordnet.

Um im Wartungsfalle einen einfachen Zugriff zu den UV-Lampen zu erhalten, wird gemäß der Erfindung vorgesehen, daß die Liegeplatte und/oder die Abdeckplatte mit ihrer einen Seitenkante in eine an der Liege, vorzugsweise um eine Längsachse schwenkbare Halteschiene einsteckbar und dort arretierbar ist. Damit ist es möglich, mit wenigen Handgriffen die Liegeplatte und/oder die Abdeckplatte an der Liege hochzuschwenken, um zu den UV-Lampen zu gelangen.

Die Abdeckplatte liegt zweckmäßig mit ihren Seitenrändern auf an der Liege angeordneten Widerlagerflächen dichtend auf und ist dort lösbar befestigt. Um einen dichten Abschluß zu erhalten, kann die Abdeckplatte im Bereich der Widerlagerflächen flanschartig geformt werden. Grundsätzlich ist es möglich, auch den Zwischenbereich zwischen Abdeckplatte und Liegeplatte mit Kühlluft zu beaufschlagen, um eine verbesserte Kühlung der Liegeplatte zu erhalten.

Gemäß einer weiteren bevorzugten Ausgestaltung der Erfindung weist die Liegeplatte eine nach unten gebogene, einen Randwulst der Liege formschlüssig umgreifende Randpartie auf. Der Randwulst kann dabei aus einem elastomeren, nachgiebigen Material, vorzugsweise aus Polyurethanschaumstoff, bestehen oder mit einem solchen aufgefüttert sein. Um den Ein- und Ausstieg zu erleichtern, weisen die Randpartie der Liegeplatte und der Randwulst der Liege eine schräg zum Außenrand hin abfallende Einstiegzone auf. Eine weitere Verbesserung in dieser Hinsicht wird dadurch erzielt, daß die Randpartie und der Randwulst einen in Längsrichtung konvex geschwungenen Außenrand bilden. Eine zuverlässige Verankerung der Liegeplatte an der Liege wird dadurch erzielt, daß die Randpartie der Liegeplatte den Außenrand des Randwulsts formschlüssig umfaßt und nach unten hin unter einem gegenüber der Liegefläche spitzen Winkel hintergreift.

Im folgenden wird die Erfindung anhand eines in der Zeichnung in schematischer Weise dargestellten Ausführungsbeispiels näher erläutert. Es zeigen
- Fig. 1: xeine schaubildliche Darstellung eines Bestrahlungsgeräts für die Ganzkörperbestrahlung;
- Fig. 2: die Liege des Bestrahlungsgeräts nach Fig. 1 in schaubildlicher Darstellung;
- Fig. 3: eine Stirnseitenansicht der Liege nach Fig. 1;
- Fig. 4: einen Querschnitt durch die Liege nach Fig. 1;
- Fig. 5: die transparente Liegeplatte der Liege nach Fig. 1 in schaubildlicher Darstellung.

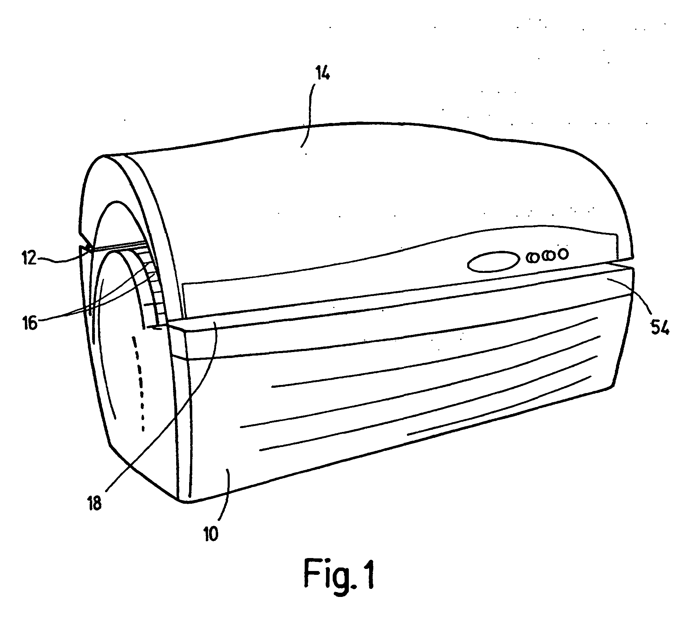

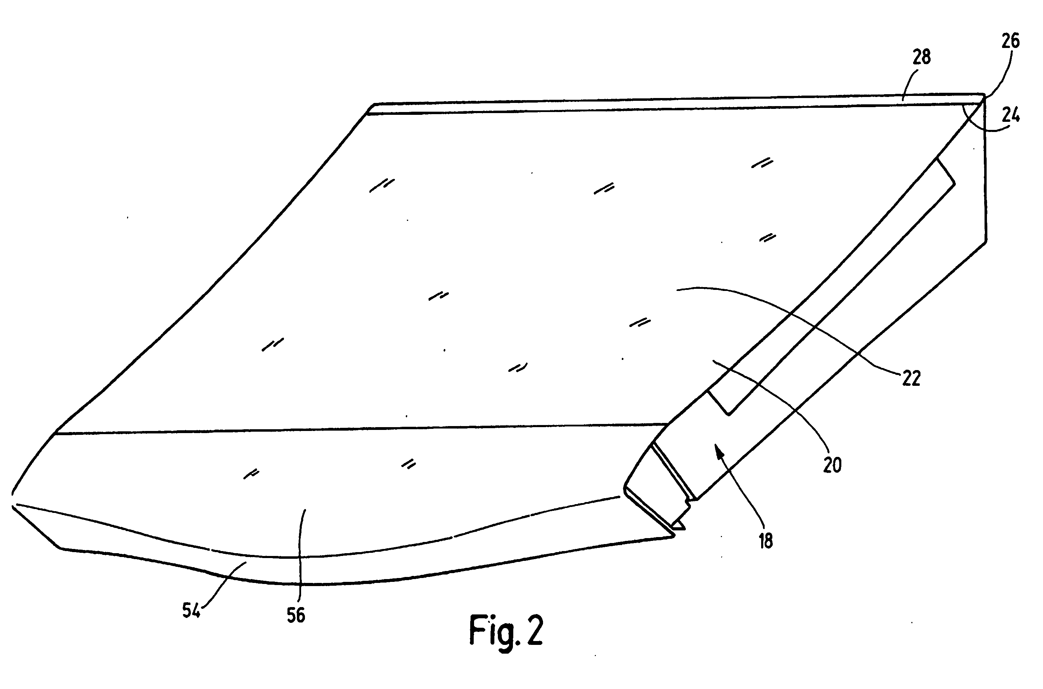

Das in Fig. 1 dargestellte Bestrahlungsgerät ist als Solarium für die Ganzkörperbräunung bestimmt. Es besteht aus einem auf dem Boden aufstehenden Unterteil 10, einem gegenüber dem Unterteil um eine rückwärtige Horizontalachse 12 verschwenkbaren Oberteil 14, die beide mit als Leuchtstoffröhren ausgebildeten UV-Lampen 16 bestückt sind. Das Unterteil 10 enthält eine Liege 18, die in den Fig. 2 bis 5 als solche und in ihren Bestandteilen dargestellt ist.

Die Liege 18 weist eine transparente Liegeplatte 20 aus mattiertem Acrylglas auf, die an ihrer Oberfläche eine muldenartige Liegefläche 22 bildet und die mit ihrem hinteren Seitenrand 24 in eine an der Liege 18 gegenüber der Liege um eine horizontale Längsachse 26 verschwenkbare Halteschiene 28 eingesteckt und an dieser lösbar festgeklemmt ist.

Weiter sind in der Liege 18 die als Leuchtstoffröhren ausgebildeten UV-Lampen 16 so angeordnet, daß sie über je einen Reflektor 30 nach oben in Richtung Liegeplatte 20 strahlen. Zwischen der Liegeplatte 20 und den UV-Lampen 16 befindet sich außerdem eine transparente Abdeckplatte 32, die ebenfalls aus mattiertem Acrylglas bestehen kann und die einen Kühlluftkanal 34 für die UV-Lampen 16 begrenzt. Die Abdeckplatte weist eine quer zur Längserstreckung der Liege wellige Gestalt auf mit nach unten und nach oben weisenden Wellenbögen 36,38. Sie stützt sich mit ihren unteren Wellenbögen 36 an aus Strangpreßprofilen bestehenden Widerlagerstegen 40 ab und liegt mit ihren flanschartigen Seitenrändern 42 an Widerlagerflächen 44 der Liege 18 dichtend auf. Im vorderen Seitenrandbereich ist die Abdeckplatte 32 mittels Schrauben 46 an der Liege 18 lösbar befestigt.

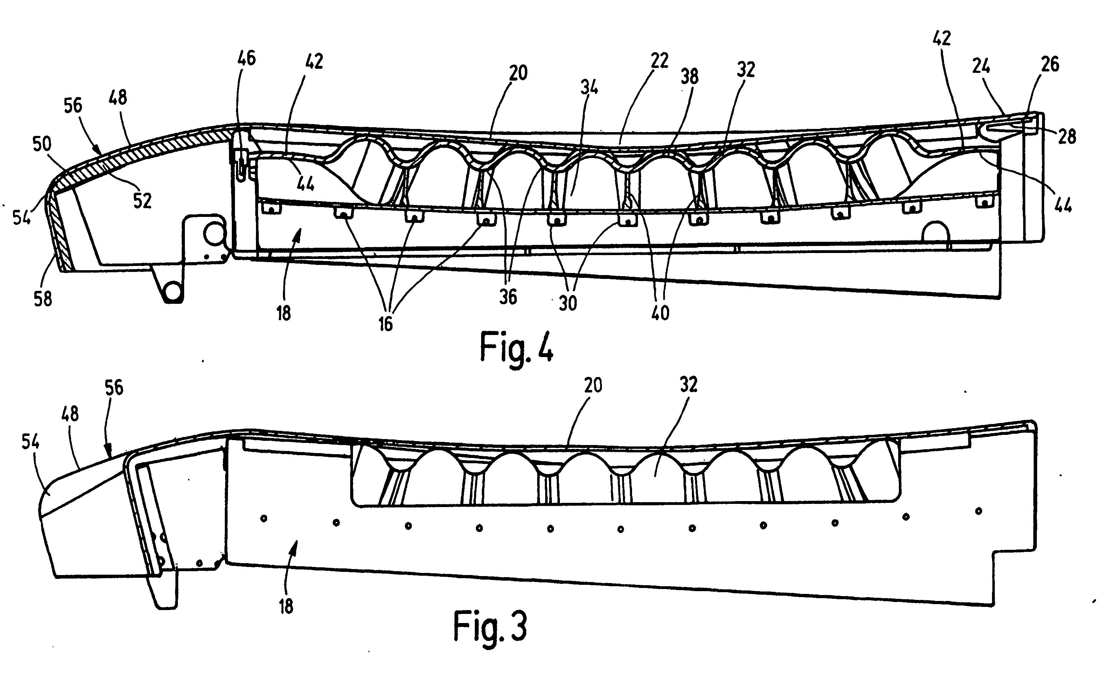

Wie aus Fig. 3 und 4 zu ersehen ist, liegt die Liegeplatte 20 mit ihrer Unterseite auf den oberen Wellenbögen 38 der Abdeckplatte 32 auf. Dadurch wird erreicht, daß im lichtdurchfluteten Bereich keine störenden Stützelemente angeordnet sind, die zu einer ungleichmäßigen Bräunung führen könnten.

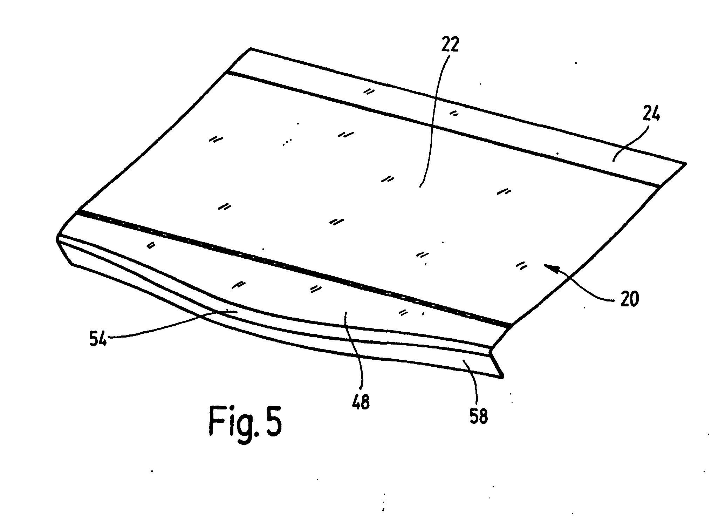

Auf der Einstiegsseite des Solariums weist die Liegeplatte 20 eine nach unten gebogene Randpartie 48 auf, die einen Randwulst 50 der Liege 18 formschlüssig umgreift. Der Randwulst 50 ist durch eine Auflage 52 aus elastomerem, elastisch nachgiebigem Material, vorzugsweise aus Polyurethanschaumstoff, aufgefüttert. Wie aus Fig. 2 zu erkennen ist, bilden die Randpartie 48 der Liegeplatte 20 und der Randwulst 50 der Liege 18 eine schräg zum Außenrand 54 hin abfallende Einstiegszone 56. Der Außenrand 54 ist dabei in Längsrichtung der Liege 18 konvex geschwungen (vgl. Fig. 2 und 5). Die Randpartie 48 der Liegeplatte 20 umfaßt den Außenrand 54 des Randwulsts 50 formschlüssig und untergreift diesen in seinem unteren Teil 58 unter einem spitzen Winkel gegenüber der Liegefläche 22.

Um zu Wartungszwecken an die UV-Lampen 16 innerhalb der Liege heranzukommen, muß die Randpartie 48 über den Randwulst 50 nach oben gezogen und die Liegeplatte 20 sodann um die Längsachse 26 der Halteschiene 28 nach oben geschwenkt werden. Sodann kann die Abdeckplatte 32 nach Lösen der Schrauben 46 unter Freigabe des Zugangs zu den UV-Lampen 16 von der Liege abgenommen werden.

Zusammenfassend ist folgendes festzuhalten: Die Erfindung bezieht sich auf ein Bestrahlungsgerät, insbesondere auf ein Solarium zur Ganzkörperbestrahlung. Das Bestrahlungsgerät weist eine Liege 18 auf, auf der eine an ihrer Oberfläche eine vorzugsweise muldenförmige Liegefläche 22 bildende transparente Liegeplatte 20 angeordnet ist. Weiter sind innerhalb der Liege eine Anzahl UV-Lampen 16 angeordnet, die nach oben zur Liegeplatte hin strahlen. Zwischen der Liegeplatte 20 und den UV-Lampen 16 befindet sich eine transparente Abdeckplatte 32, die einen Kühlluftkanal 34 für die UV-Lampen 16 begrenzt. Um eine gleichmäßige Bestrahlung zu gewährleisten, weist die Abdeckplatte 32 eine wellige Gestalt mit innerhalb der Liege nach unten und nach oben weisenden Wellenbögen 36,38 auf. Sie stützt sich zumindest mit einem Teil der unteren Wellenbögen 36 an Widerlagerflächen oder Widerlagerstegen 40 der Liege 18 ab, während die Liegeplatte 20 mit ihrer Unterseite zumindest auf einem Teil der oberen Wellenbögen 38 der Abdeckplatte 32 aufliegt.

## Patentansprüche

1. Bestrahlungsgerät, insbesondere Solarium, mit einer Liege (18), an deren Oberfläche eine vorzugsweise muldenförmige Liegefläche (22) bildenden transparenten Liegeplatte, (20) angeordnet ist, wobei die Liege (18) eine Anzahl nach oben zur Liegeplatte (20) hin strahlende UV-Lampen (16) und eine zwischen der Liegeplatte (20) und den UV-Lampen (16) angeordnete transparente Abdeckplatte (32) aufweist, die einen Kühlluftkanal (34) für die UV-Lampen (16) begrenzt, wobei die Abdeckplatte (32) eine wellige Gestalt mit nach unten und nach oben weisenden Wellenbögen (36,38) aufweist und wobei die Liegeplatte (20) mit ihrer Unterseite zumindest auf einem Teil der oberen Wellenbögen (38) der Abdeckplatte (32) aufliegt, **dadurch gekennzeichnet, dass** sich die Abdeckplatte (32) zumindest mit einem Teil ihrer unteren Wellenbögen (36) an Widerlagerflächen oder Widerlagerstegen (40) abstützt und dass die Liegeplatte (20) und/oder die Abdeckplatte (32) mit ihrem einen Seitenrand (24) in eine an der Liege (18) angebrachten, um eine Achse, vorzugsweise um eine Längsachse (26), schwenkbare Halteschiene (28) einsteckbar und dort arretierbar ist.

2. Bestrahlungsgerät nach Anspruch 1, **dadurch gekennzeichnet, dass** die Liegeplatte (20) und/oder die Abdeckplatte (32) aus Acrylglas besteht.

3. Bestrahlungsgerät nach Anspruch 1 oder 2, **dadurch gekennzeichnet, dass** die Liegeplatte (20) und/oder die Abdeckplatte (32) aus mattiertem oder aufgerautem Acrylglas besteht.

4. Bestrahlungsgerät nach einem der Ansprüche 1 bis 3, **dadurch gekennzeichnet, dass** den als Leuchtstoffröhren ausgebildeten UV-Lampen einzeln oder zu mehreren ein quer zur Längserstreckung der UV-Lampen (16) geschwungener Wellenbogen (38) zugeordnet ist.

5. Bestrahlungsgerät nach einem der Ansprüche 1 bis 4, **dadurch gekennzeichnet, dass** der Zwischenraum zwischen der Liegeplatte (20) und der Abdeckplatte (32) mit Kühlluft beaufschlagbar ist.

6. Bestrahlungsgerät nach einem der Ansprüche 1 bis 5, **dadurch gekennzeichnet, dass** die Liegeplatte (20) eine nach unten gebogene Randpartie (48) aufweist, die einen Randwulst (50) der Liege (18) formschlüssig untergreift.

7. Bestrahlungsgerät nach Anspruch 6, **dadurch gekennzeichnet, dass** der Randwulst (50) aus einem elastomeren Material, vorzugsweise aus Polyurethanschaumstoff, besteht oder mit einem solchen aufgefüttert ist.

8. Bestrahlungsgerät nach Anspruch 6 oder 7, **dadurch gekennzeichnet, dass** die Randpartie (48) der Liegeplatte (20) und der Randwulst (50) der Liege (18) eine schräg zum Außenrand (54) hin abfallende Einstiegzone (56) aufweisen.

9. Bestrahlungsgerät nach einem der Ansprüche 6 bis 8, **dadurch gekennzeichnet, dass** die Randpartie (48) und der Randwulst (50) einen in Längsrichtung der Liegefläche (22) konvex geschwungenen Außenrand (54) aufweisen.

10. Bestrahlungsgerät nach einem der Ansprüche 6 bis 9, **dadurch gekennzeichnet, dass** die Randpartie (48) der Liegeplatte (20) den Außenrand (54) des Randwulsts (50) formschlüssig umfasst und nach unten hin unter spitzem Winkel gegenüber der Liegefläche (22) hintergreift.

11. Bestrahlungsgerät nach einem der Ansprüche 1 bis 10, **dadurch gekennzeichnet, dass** die Liegeplatte (20) und/oder die Abdeckplatte (32) im Bereich ihrer fuß- und kopfseitigen Enden undurchsichtig mattiert oder bedruckt sind.

12. Bestrahlungsgerät nach einem der Ansprüche 1 bis 11, **dadurch gekennzeichnet, dass** die Abdeckplatte (32) mit ihren Seitenrändern (42) auf an der Liege (18) angeordneten Widerlagerflächen (44) dichtend anliegt und dort lösbar befestigt ist.

## Claims

1. Irradiation apparatus, in particular solarium, with a couch (18), at the surface of which a transparent reclining panel (20), forming a preferably trough-shaped reclining area (22), is disposed, wherein the couch (18) has a number of UV lamps (16), which radiate upwards towards the reclining panel (20), and a transparent covering panel (32), which is disposed between the reclining panel (20) and the UV lamps (16) and defines a cooling air duct (34) for the UV lamps (16), wherein the covering panel (32) is formed in an undulating manner with downward and upward pointing undulating arcs (36, 38), and wherein the reclining panel (20) lies with its underside at least on some of the upper undulating arcs (38) of the covering panel (32), **characterised in that** the covering panel (32) is supported at least with some of its lower undulating arcs (36) at abutment areas or abutment webs (40), and that the reclining panel (20) and/or the covering panel (32) can be inserted with one side edge (24) thereof in a retaining rail (28), which is attached to the couch (18) and is pivotable about an axis, preferably about a longitudinal axis (26), and locked here.

2. Irradiation apparatus according to Claim 1, **characterised in that** the reclining panel (20) and/or the covering panel (32) consist(s) of acrylic glass.

3. Irradiation apparatus according to Claim 1 or 2, **characterised in that** the reclining panel (20) and/or the covering panel (32) consist(s) of mat-finished or roughened acrylic glass.

4. Irradiation apparatus according to any one of Claims 1 to 3, **characterised in that** an undulating arc (38), curved transversely to the longitudinal extent of the UV lamps (16), is associated with the UV lamps, which are formed as fluorescent tubes, individually or in a plurality.

5. Irradiation apparatus according to any one of Claims 1 to 4, **characterised in that** cooling air can be applied to the interspace between the reclining panel (20) and the covering panel (32).

6. Irradiation apparatus according to any one of Claims 1 to 5, **characterised in that** the reclining panel (20) has a downward bent edge part (48) which positively engages under an edge bead (50) of the couch (18).

7. Irradiation apparatus according to Claim 6, **characterised in that** the edge bead (50) consists of an elastomeric, material, preferably of polyurethane foam, or is lined with a material of this kind.

8. Irradiation apparatus according to Claim 6 or 7, **characterised in that** the edge part (48) of the reclining panel (20) and the edge bead (50) of the couch (18) have an entry zone (56) which slopes downwards towards the outer edge (54).

9. Irradiation apparatus according to any one of Claims 6 to 8, **characterised in that** the edge part (48) and the edge bead (50) have an outer edge (54) which is convexly curved in the longitudinal direction of the reclining area (22).

10. Irradiation apparatus according to any one of Claims 6 to 9, **characterised in that** the edge part (48) of the reclining panel (20) positively encompasses the outer edge (54) of the edge bead (50) and engages downwards behind this at an acute angle with respect to the reclining area (22).

11. Irradiation apparatus according to any one of Claims 1 to 10, **characterised in that** the reclining panel (20) and/or the covering panel (32) are mat-finished or printed so as to be opaque in the region of their foot and head ends.

12. Irradiation apparatus according to any one of Claims 1 to 11, **characterised in that** the covering panel (32) lies in a sealing manner with its side edges (42) on abutment areas (44) disposed at the couch (18) and is fastened here such that it can be released.

## Revendications

1. Appareil d'irradiation, notamment solarium muni d'un plan de couchage (18) à la face supérieure duquel se trouve un panneau transparent de couchage (20) matérialisant une surface de couchage (22) de préférence en forme d'auge, ledit plan de couchage (18) présentant un certain nombre de lampes (16) à UV irradiant vers le haut, en direction du panneau de couchage (20), et une plaque transparente de recouvrement (32) qui est interposée entre le panneau de couchage (20) et les lampes (16) à UV, et délimite un canal (34) à air de refroidissement destiné auxdites lampes (16) à UV, sachant que la plaque de recouvrement (32) possède une configuration ondulée comportant des arceaux (36, 38) orientés vers le bas et vers le haut, et sachant que le panneau de couchage (20) repose, par sa face inférieure, au moins sur une partie des arceaux supérieurs (38) de ladite plaque de recouvrement (32), **caractérisé par le fait que** la plaque de recouvrement (32) prend appui, au moins par une partie de ses arceaux inférieurs (36), sur des surfaces de contre-appui ou des membrures de soutien (40) ; et **par le fait que** le panneau de couchage (20) et/ou la plaque de recouvrement (32) peut être emboîté(e), par son bord latéral (24), dans une glissière d'arrêt (28) installée sur le plan de couchage (18) et pouvant pivoter autour d'un axe, de préférence autour d'un axe longitudinal (26), et peut être consigné(e) à demeure sur ladite glissière.

2. Appareil d'irradiation selon la revendication 1, **caractérisé par le fait que** le panneau de couchage (20) et/ou la plaque de recouvrement (32) consiste(nt) en du verre acrylique.

3. Appareil d'irradiation selon la revendication 1 ou 2, **caractérisé par le fait que** le panneau de couchage (20) et/ou la plaque de recouvrement (32) consiste(nt) en du verre acrylique dépoli ou rendu rugueux.

4. Appareil d'irradiation selon l'une des revendications 1 à 3, **caractérisé par le fait que** des arceaux (38), sinuant transversalement par rapport à l'étendue longitudinale des lampes (16) à UV, sont affectés, individuellement ou à plusieurs, auxdites lampes à UV réalisées sous la forme de tubes fluorescents.

5. Appareil d'irradiation selon l'une des revendications 1 à 4, **caractérisé par le fait que** l'espace intercalaire, situé entre le panneau de couchage (20) et la plaque de recouvrement (32), peut être sollicité par de l'air de refroidissement.

6. Appareil d'irradiation selon l'une des revendications 1 à 5, **caractérisé par le fait que** le panneau de couchage (20) présente une partie marginale (48) coudée vers le bas et emprisonnant par en bas, avec complémentarité de formes, un renflement marginal (50) du plan de couchage (18).

7. Appareil d'irradiation selon la revendication 6, **caractérisé par le fait que** le renflement marginal (50) est constitué d'un matériau élastomère, de préférence d'une mousse de polyuréthane, ou bien est garni d'un tel matériau.

8. Appareil d'irradiation selon la revendication 6 ou 7, **caractérisé par le fait que** la partie marginale (48) du panneau de couchage (20), et le renflement marginal (50) du plan de couchage (18), comportent une zone d'accès (56) déclinant à l'oblique vers le bord extérieur (54).

9. Appareil d'irradiation selon l'une des revendications 6 à 8, **caractérisé par le fait que** la partie marginale (48) et le renflement marginal (50) présentent un bord extérieur (54) à incurvation convexe dans le sens longitudinal de la surface de couchage (22).

10. Appareil d'irradiation selon l'une des revendications 6 à 9, **caractérisé par le fait que** la partie marginale (48) du panneau de couchage (20) ceinture, par complémentarité de formes, le bord extérieur (54) du renflement marginal (50) et vient en prise par-derrière, vers le bas, en décrivant un angle aigu vis-à-vis de la surface de couchage (22).

11. Appareil d'irradiation selon l'une des revendications 1 à 10, **caractérisé par le fait que** le panneau de couchage (20) et/ou la plaque de recouvrement (32) présente(nt) un dépoli opaque ou une sérigraphie dans la région de ses (leurs) extrémités situées aux pieds et à la tête.

12. Appareil d'irradiation selon l'une des revendications 1 à 11, **caractérisé par le fait que** la plaque de recouvrement (32) repose de manière étanche, par ses bords latéraux (42), sur des surfaces de contre-appui (44) disposées sur le plan de couchage (18), et est fixée amoviblement auxdites surfaces.
